# EUROPÄISCHE PATENTSCHRIFT

(11) **EP 4 216 781 B1**
(45) Veröffentlichungstag und Bekanntmachung des Hinweises auf die Patenterteilung: **05.03.2025**
(21) Anmeldenummer: 22802921.1
(22) Anmeldetag: 12.10.2022
(51) Int. Cl.: A47K 13/30, A61B 5/0205, A61B 5/00

(54) **WC-SITZGARNITUR UND WC**
TOILET SEAT ASSEMBLY AND TOILET
ENSEMBLE SIÈGE DE TOILETTES ET TOILETTES

(30) Priorität: 15.12.2021 DE 102021133283
(43) Veröffentlichungstag der Anmeldung: 02.08.2023
(73) Patentinhaber: Hamberger Industriewerke GmbH, 83071 Stephanskirchen (DE)
(72) Erfinder: HAMBERGER, Peter, 83071 Stephanskirchen (DE)
(74) Vertreter: Winter, Brandl - Partnerschaft mbB
(86) Internationale Anmeldenummer: PCT/EP2022/078377
(87) Internationale Veröffentlichungsnummer: WO 2023/110187

(56) Entgegenhaltungen:
- EP-A1- 3 906 852
- DE-B4- 102016 108 379
- US-A1- 2018 303 466

## Beschreibung

Die Erfindung betrifft eine WC-Sitzgarnitur mit einer integrierten Sensorik und ein mit einer derartigen WC-Sitzgarnitur ausgeführtes WC.

Eine gattungsgemäße WC-Sitzgarnitur ist in der auf die Anmelderin zurückgehenden Druckschrift DE 10 2016 108 379 A1 beschrieben. Eine derartige WC-Sitzgarnitur hat einen WC-Sitz, in den eine Sensorik zur mittelbaren oder unmittelbaren Erfassung physiologischer oder gesundheitsspezifischer Parameter/Kenngrößen eines Nutzers/einer Nutzerin - im Folgenden "Nutzer" genannt - integriert ist. Diese Parameter/Kenngrößen werden in der Literatur auch als Vitalparameter bezeichnet. Zur Verarbeitung der über die Sensorik erfassten Messwerte ist eine Dockingstation vorgesehen, an die die WC-Sitzgarnitur angesetzt ist und in der eine Steuereinheit zur Ansteuerung der Sensorik und zur Erfassung der von der Sensorik erfassten Daten aufgenommen ist. Die Technikbox hat des Weiteren eine Kommunikationsschnittstelle, über die die Daten an eine Auswerteeinheit, beispielsweise ein Smartphone, übertragen werden können. Diese Daten können dann zur Information des Nutzers oder weiterer interessierter Personen ausgelesen werden.

In der Druckschrift US 10,292,658 B2 ist eine WC-Sitzgarnitur beschrieben, in die eine Sensorik zur Erfassung des Blutdrucks, des Schlagvolumens und der Blutoxygenierung und auch anderer Vitalparameter, wie beispielsweise das Gewicht und der Puls des Nutzers integriert ist.

Ein ähnliches Konzept ist in der DE 10 2018 102 585 A1 beschrieben, wobei ebenfalls in eine WC-Sitzgarnitur Sensoren zur Erfassung von für die Körperfunktion repräsentativen Signalen (Vitalparameter) integriert sind. Diese Sensoren können beispielsweise Elektroden zur Erfassung von Potentialdifferenzen, insbesondere zur Erfassung eines EKGs zwischen Extremitäten des Nutzers sein, so dass kritische Zustände, wie beispielsweise ein Vorhofflimmern, erkannt werden können.

Derartige medizinische WC-Sitzgarnituren sind sowohl hinsichtlich der Sensorik als auch der Auswerteeinheiten zur Verarbeitung der Messsignale für den speziellen Anwendungsbereich optimiert, wobei eine umfassende Kontrolle von Vitalparametern und eine dynamische Auswertung unter Berücksichtigung auch neuester Analyse- und Therapiemethoden nicht möglich ist.

In der nachveröffentlichten DE 10 2021 130 834.8 ist eine äußerst weit entwickelte WC-Sitzgarnitur beschrieben, in der eine Sensorik sowie Kommunikationsschnittstellen und Auswerte-/Diagnoseeinheiten integriert sind, über die die mittels der Sensorik erfassten Daten ausgewertet werden können und entsprechende Empfehlungen an den Nutzer oder Dritte abgegeben werden.

Die dabei verwendete Sensorik ist äußerst komplex, so dass ein erheblicher Aufwand erforderlich ist, um diese Sensorik in die WC-Sitzgarnitur zu integrieren.

Weiterer Stand der Technik kann der EP 3 906 852 A1 und der US 2018/0303466 A1 entnommen werden.

Demgegenüber liegt der Erfindung die Aufgabe zugrunde, eine WC-Sitzgarnitur und ein mit einer derartigen WC-Sitzgarnitur versehenes WC zu schaffen, die eine vergleichsweise einfache Auswertung/Erfassung von Vitalparametern ermöglicht.

Diese Aufgabe wird im Hinblick auf die WC-Sitzgarnitur durch die Merkmale des Patentanspruchs 1 und im Hinblick auf das WC durch die Merkmale des nebengeordneten Patentanspruchs 7 gelöst.

Vorteilhafte Weiterbildungen der Erfindung sind Gegenstand der Unteransprüche.

Die erfindungsgemäße WC-Sitzgarnitur hat Einrichtungen, zur Erfassung von Vitalparametern und eine Technikbox, in der eine Steuereinheit zur Steuerung der Einrichtungen aufgenommen ist. Die Technikbox enthält des Weiteren Komponenten zur individualisierten Erfassung von über die Einrichtungen erfassten Daten, insbesondere von Vitalparametern. Die erfindungsgemäße WC-Sitzgarnitur ist des Weiteren mit einer Kommunikationsschnittstelle zum Empfangen und Senden von Daten ausgeführt, wobei diese Kommunikationsschnittstelle in Datenverbindung mit einer Auswerteeinheit steht. Diese ist vorzugsweise mit einer Diagnoseeinheit ausgeführt, die ausgelegt ist, in Abhängigkeit von den verarbeiteten Daten Hinweise oder Empfehlungen an den Nutzer oder an Dritte auszugeben. Erfindungsgemäß ist eine der Einrichtungen eine Kamera, über die der Stuhlgang oder der Urin erfassbar ist. Die Kamera ist in Richtung eines Bodens einer Keramik ausgerichtet, so dass während der Nutzung der Toilette der Stuhlgang und der Urin, ggf. auch der Volumenstrom des Urinstrahls erfasst werden kann. Außerdem ist die Kamera in den WC-Sitz integriert, so dass die ästhetische Anmutung der WC-Sitzgarnitur nicht durch die Kamera gestört ist und es auch für Dritte nicht ersichtlich ist, dass die WC-Sitzgarnitur mit einer Kamera ausgeführt ist. Die Kamera ist mit dem WC-Sitz verpresst.

Derartige Kameras sind vergleichsweise preisgünstig und ermöglichen es, durch Bildanalyse des Stuhlgangs und/oder des Urins auf den Gesundheitszustand des Nutzers zurückzuschließen.

Die Kamera ist in Richtung auf die Keramik ausgerichtet, so dass Körperteile des Nutzers nicht erfasst werden.

Bei einem Ausführungsbeispiel der Erfindung ist die Kamera in eine Aufnahme des WC-Sitzes eingesetzt.

Eine besonders einfache Positioniermöglichkeit ist gegeben, wenn die WC-Sitzgarnitur über zwei WC-Sitzgelenke verfügt, über die die WC-Sitzgarnitur mit einer Keramik verbindbar ist.

Eine Nachrüstung bestehender WC-Sitzgarnituren ist möglich, wenn die Kamera in ein an die WC-Sitzgarnitur angesetztes Rückbrett integriert ist. Zur Auswertung der Bilddaten ist vorzugsweise die Auswerteeinheit mit einer Bildverarbeitungssoftware ausgeführt.

Bei einem Ausführungsbeispiel der Erfindung ist die WC-Sitzgarnitur mit einer Aktivierungseinheit ausgeführt, so dass die Kamera nur durch den Nutzer oder sonstige befugte Personen aktiviert werden kann.

Bei einem bevorzugten Ausführungsbeispiel der Erfindung ist die Einrichtung des Weiteren mit Sensoren zum Erfassen eines EKGs, der Körpertemperatur, der Sauerstoffsättigung im Blut, einer Pulsfrequenz oder dergleichen ausgeführt.

Die Auswertung der Vitalparameter und der über die Kamera erfassten Daten ist besonders zuverlässig, wenn die Auswerteeinheit und/oder die Steuereinheit eine Eingabeeinrichtung zum Eingeben von Individualdaten eines oder mehrerer Nutzer aufweist, so dass die erfassten Daten dem jeweiligen Nutzer zugeordnet werden können.

Dabei wird es bevorzugt, wenn die WC-Sitzgarnitur auch mit einem entsprechenden Individualspeicher ausgeführt ist.

Die Eingabe dieser Daten und die Datenübertragung zwischen den einzelnen Schnittstellen des Systems erfolgt vorzugsweise drahtlos.

Bevorzugte Ausführungsbeispiele der Erfindung werden im Folgenden anhand schematischer Zeichnung näher erläutert. Es zeigt:

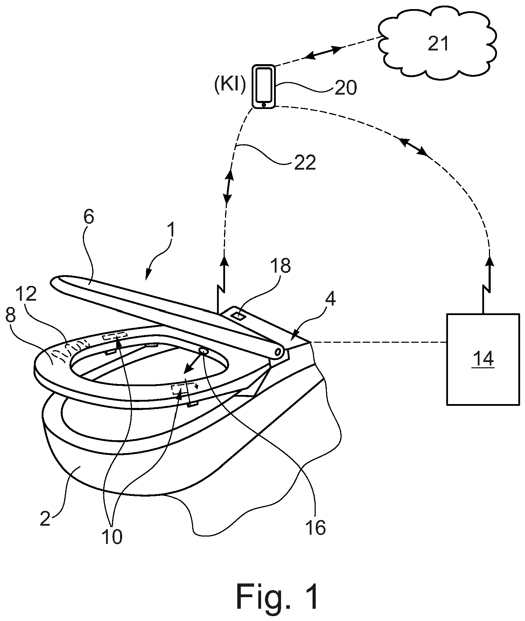

Figur 1 eine dreidimensionale stark vereinfachte Prinzipdarstellung einer erfindungsgemäßen WC-Sitzgarnitur.

Figur 1 zeigt eine schematisierte 3D-Ansicht einer WC-Sitzgarnitur 1, die auf einer Keramik 2 abgestützt ist. Diese WC-Sitzgarnitur 1 ist über eine Sitzgelenkanordnung an einer Technikbox 4 angelenkt, die mit der Keramik 2 verankert ist. Beim dargestellten Ausführungsbeispiel hat die WC-Sitzgarnitur einen WC-Deckel 6 und einen WC-Sitz 8, wobei in letzterem eine Sensorik 10 integriert ist, die die Messung von Vitalparametern ermöglicht. Zusätzlich ist der WC-Sitz 8, wie in Figur 1 angedeutet, mit einer Sitzheizung 12 oder sonstigen Funktionselementen ausgeführt sein. Die Stromversorgung der Sitzheizung 12 und der Sensorik 10 erfolgt beispielsweise über eine in die Technikbox 4 integrierte Stromquelle, beispielsweise einen Akku. Selbstverständlich kann die Stromversorgung jedoch auch in herkömmlicher Weise über eine 230V-Stromversorgung erfolgen.

Die Sensorik 10 ist so ausgelegt, dass neben einem EKG auch weitere Vitalparameter, wie beispielsweise der Puls, die Körpertemperatur, die Sauerstoffsättigung, etc. erfasst werden können. Des Weiteren kann eine zusätzliche Station, beispielsweise eine Bluetooth-Waage 14 vorgesehen sein, um das Gewicht oder sonstige zusätzliche Vitalparameter zu erfassen. Diese zusätzliche Station 14 steht ebenfalls in Datenverbindung mit der Technikbox 4 oder einer im Folgenden noch näher beschriebenen Auswerteeinheit 20.

Erfindungsgemäß ist des Weiteren eine Kamera 16 vorgesehen. Diese ist derart in Richtung des Bodens der Keramik 2 ausgerichtet, so dass während der Nutzung der Toilette der Stuhlgang und der Urin, ggf. auch der Volumenstrom des Urinstrahls erfasst werden kann.

Diese Kamera 16 kann beispielsweise als Fotokamera oder aber auch Filmkamera ausgeführt sein, so dass eine kontinuierliche oder diskontinuierliche Erfassung der Ausscheidungen ermöglicht ist. Die Kamera 16 ist in den WC-Sitz 8 integriert. Die Kamera 16 ist beim Pressen des Sitzes integriert. Bei einer zweischaligen Sitzkonstruktion kann die Kamera 16 in die Schalenkonstruktion eingesetzt sein.

Es ist weiterhin denkbar, dass die Kamera 10 im Bereich der Technikbox 4 angeordnet ist, wobei die Positionierung so erfolgt, dass die Blickrichtung in den Aufnahmeraum der Keramik gewährleistet ist.

Die Technikbox 4 kann mit einer Auswerteeinheit zur Auswertung der Bildinformationen ausgeführt sein. Die Technikbox 4 hat jedoch auch eine Kommunikationsschnittstelle (hierauf wird unten eingegangen), über die die Bildinformationen an eine externe Auswerteeinheit übermittelt werden. Diese integrierte oder externe Auswerteeinheit kann mit einer Diagnoseeinheit ausgeführt sein, die ausgelegt ist, die Bildinformationen im Hinblick auf Vitalparameter und Parameter zur Definition des Gesundheitszustands des Nutzers zu erstellen und auszuwerten. So ist es beispielweise möglich, aus dieser Auswertung, möglicherweise in Kombination mit sonstigen Sensoren, Blutzuckerunregelmäßigkeiten, Darmerkrankungen, Flüssigkeitsmangel, sonstige Mangelerscheinungen, eine Fehlernährung, Stoffwechselerkrankungen etc. zu erkennen.

Die WC-Sitzgarnitur kann des Weiteren einen Sensor zur Erfassung einer Nutzung der WC-Sitzgarnitur aufweisen.

Dadurch ist es beispielsweise möglich, die Stromversorgung nur dann zuzuschalten, wenn die WC-Sitzgarnitur benutzt wird. Des Weiteren kann noch eine Anzeige 18 vorgesehen sein, über die der Nutzer über den Betriebszustand informiert ist oder aber Hinweise über erfasste Vitalparameter oder Diagnosedaten erhält.

Die über die Sensorik 10 und die Kamera 16 erfassten Messsignale können über ein Funkmodul oder aber auch kabelgebunden an die externe Auswerteeinheit 20 übertragen werden, die wiederum mit einer Cloud 21 oder internetbasierten Datenbanken in Datenverbindung steht. In der Figur 1 ist eine Variante angedeutet, bei der die Auswerteeinheit 20 als Tablet ausgeführt ist, das die Messsignale 22 über Funk, beispielsweise WLAN oder Bluetooth oder über eine NFC-Technologie oder dergleichen empfängt. In dieser Auswerteeinheit 20 ist dann beispielsweise eine mit KI ausgeführte Auswertesoftware gespeichert, über die die Messsignale 22 mit Hilfe der in der Cloud 20 abgespeicherten Datenbanken auswertet und dann zur Information des Nutzers oder weiterer interessierter Personen oder einer das WC betreibenden Unity ausgelesen werden.

Durch die kombinierte Verwendung der Kamera und der sonstigen Sensorik ist somit eine umfassende Überprüfung von Vitalparametern und damit des Gesundheitszustandes des Nutzers möglich.

Die Kamera wird vorzugsweise so integriert, dass deren Anwesenheit nicht erkennbar ist. Die Betriebssicherheit und auch die Datensicherheit werden optimiert, wenn die WC-Sitzgarnitur 1 mit einer Einrichtung, beispielsweis einer Sensorik zur Erfassung eines individuellen Nutzers versehen ist, so dass die Kamera nur bei bestimmten Nutzern aktiviert wird. Alternativ kann eine Aktivierungseinheit vorgesehen sein, die zur Aktivierung der Kamera gezielt betätigt werden muss.

Die Kamera ist wie in Figur 1 dargestellt, in den WC-Sitz 8 integriert.

Offenbart ist eine WC-Sitzgarnitur mit einer integrierten Kamera.

### Bezugszeichenliste:

- 1: WC-Sitzgarnitur
- 2: Keramik/WC
- 4: Technikbox
- 6: WC-Deckel
- 8: WC-Sitz
- 10: Sensorik
- 12: Sitzheizung
- 14: Station
- 16: Kamera
- 18: Anzeige
- 20: Auswerteeinheit
- 21: Cloud
- 22: Messsignal

## Patentansprüche

1. WC-Sitzgarnitur mit Einrichtungen zur Erfassung von Vitalparametern oder sonstigen Daten, wobei die WC-Sitzgarnitur eine Technikbox (4) aufweist, in der eine Steuereinheit zur Steuerung der Einrichtungen und zur individualisierten Erfassung der von den Einrichtungen erfassten Daten, insbesondere Vitalparametern, und eine Kommunikationsschnittstelle zum Empfangen und Senden von Daten aufgenommen ist, wobei die Kommunikationsschnittstelle in Datenverbindung mit einer Auswerteeinheit (20) steht, eine der Einrichtungen eine Kamera (16) ist und diese in Richtung eines Bodens einer Keramik (2) ausgerichtet ist, so dass während der Nutzung der Toilette der Stuhlgang und der Urin, ggf. auch der Volumenstrom des Urinstrahls erfasst werden kann, **dadurch gekennzeichnet, dass** die Kamera (16) in einen WC-Sitz (8) integriert ist und die Kamera (16) mit dem WC-Sitz (8) verpresst ist.

2. WC-Sitzgarnitur nach Patentanspruch 1, wobei die integrierte oder externe Auswerteeinheit (20) mit einer Bildverarbeitungssoftware ausgeführt ist.

3. WC-Sitzgarnitur nach einem der vorhergehenden Patentansprüche, mit einer Aktivierungseinheit zur Aktivierung einer Kamerafunktion.

4. WC-Sitzgarnitur nach einem der vorhergehenden Patentansprüche, wobei die Einrichtungen Sensoren zum Erfassen eines EKGs, der Körpertemperatur, der Sauerstoffsättigung im But, einer Pulsfrequenz oder dergleichen aufweisen.

5. WC-Sitzgarnitur nach einem der vorhergehenden Patentansprüche, wobei die Auswerteeinheit (20) und/oder die Steuereinheit eine Eingabeeinrichtung zum Eingeben von Individualdaten eines oder mehrerer Nutzer aufweist.

6. WC-Sitzgarnitur nach Patentanspruch 5, mit einem Individualspeicher zur Speicherung der resultierenden Individualdaten eines Nutzers.

7. WC mit einer WC-Sitzgarnitur nach einem der vorhergehenden Patentansprüche.

## Claims

1. Toilet seat set comprising devices for recording vital parameters or other data, the toilet seat set, wherein the toilet seat comprises a technology box (4) in which a control unit for controlling the devices and for individualized acquisition of the data acquired by the devices, in particular vital parameters, and a communication interface for receiving and sending data is accommodated, wherein the communication interface is in data connection with an evaluation unit (20), one of the devices is a camera (16) and the camera is arranged in the direction of a base of a ceramic (2), so that during use of the toilet the bowel movement and the urine, and possibly also the volume flow of the urine stream, can be acquired, **characterized in that** the camera (16) is integrated in a toilet seat (8) and the camera (16) is pressed with the toilet seat (8).

2. Toilet seat set according to claim 1, wherein the integrated or external evaluation unit (20) is performed with an image processing software.

3. Toilet seat set according to one of the preceding patent claims, having an activation unit for activating a camera function.

4. Toilet seat set according to one of the preceding patent claims, wherein the devices comprise sensors for acquiring an ECG, the body temperature, the oxygen saturation in the blood, a pulse rate or the like.

5. Toilet seat set according to one of the preceding patent claims, wherein the evaluation unit (20) and/or the control unit comprises an input device for entering individual data of one or more users.

6. Toilet seat set according to claim 5, comprising an individual memory for storing the resulting individual data of a user.

7. Toilet with a toilet seat set according to one of the preceding claims.

## Revendications

1. Garniture de siège de WC avec des appareils pour la saisie de paramètres vitaux ou d'autres données, dans laquelle la garniture de siège de WC présente un boîtier technique (4) dans lequel un dispositif de commande est logé pour la commande des appareils et pour la saisie individualisée des données saisies par les appareils, en particulier des paramètres vitaux, et une interface de communication pour la réception et l'émission de données, dans laquelle l'interface de communication est en liaison de données avec une unité d'évaluation (20), l'un des appareils est une caméra (16) et celle-ci est orientée en direction d'un fond d'une céramique (2), de sorte que, pendant l'utilisation des toilettes, les selles et l'urine, le cas échéant le débit volumétrique du jet d'urine, peuvent également être détectés, **caractérisée en ce que** la caméra (16) est intégrée dans un siège de WC (8) et la caméra (16) est pressée avec le siège de WC (8).

2. Garniture de siège de WC selon la revendication 1, dans laquelle l'unité d'évaluation intégrée ou externe (20) est réalisée avec un logiciel de traitement d'images.

3. Garniture de siège de WC selon l'une quelconque des revendications précédentes, avec une unité d'activation pour activer une fonction de caméra.

4. Garniture de siège de WC selon l'une quelconque des revendications précédentes, dans laquelle les appareils présentent des capteurs pour détecter un ECG, la température corporelle, la saturation en oxygène dans le sang, une fréquence de pouls ou similaire.

5. Garniture de siège de WC selon l'une quelconque des revendications précédentes, dans laquelle l'unité d'évaluation (20) et/ou le dispositif de commande présente un appareil de saisie pour saisir des données individuelles d'un ou de plusieurs utilisateurs.

6. Garniture de siège de WC selon la revendication 5, avec une mémoire individuelle pour enregistrer les données individuelles résultantes d'un utilisateur.

7. WC avec une garniture de siège de WC selon l'une quelconque des revendications précédentes.
